# EUROPÄISCHE PATENTSCHRIFT

(11) **EP 3 145 458 B1**
(45) Veröffentlichungstag und Bekanntmachung des Hinweises auf die Patenterteilung: **09.08.2023**
(21) Anmeldenummer: 15734554.7
(22) Anmeldetag: 13.05.2015
(51) Int. Cl.: A61F 9/008, A61B 3/113

(54) **VORRICHTUNG ZUR BESTIMMUNG DER AUSRICHTUNG DES AUGES BEI AUGENOPERATIONEN**
DEVICE FOR DETERMINING THE ORIENTATION OF THE EYE DURING EYE SURGERIES
DISPOSITIF DE DÉTERMINATION DE L'ORIENTATION DE L'OEIL EN CAS D'OPÉRATION DES YEUX

(30) Priorität: 19.05.2014 DE 102014106993
(43) Veröffentlichungstag der Anmeldung: 29.03.2017
(73) Patentinhaber: Chronos Vision GmbH, 12247 Berlin (DE)
(72) Erfinder: SPASOVSKI, Saso, 13088 Berlin (DE); JUST, Kai, 53129 Bonn (DE); POGADE, Wolfgang, 12247 Berlin (DE)
(74) Vertreter: Willems, Volker
(86) Internationale Anmeldenummer: PCT/DE2015/000242
(87) Internationale Veröffentlichungsnummer: WO 2015/176699

(56) Entgegenhaltungen:
- WO-A1-2009/135084
- WO-A2-99/23936
- DE-A1-102012 008 998
- KR-A- 20140 008 457

## Beschreibung

Die Erfindung betrifft eine Vorrichtung zur Bestimmung der Ausrichtung des Auges bei Augenoperationen, ein Operationsmikroskop, ein Computerprogramm zur Steuerung eines Augenchirurgiesystems, sowie eine Steuereinheit für Augenchirurgiesysteme.

Für Patienten, die an einer häufig altersbedingten Opazität der natürlichen Augenlinse leiden, Grauer Star oder Katarakt genannt, verbleibt oft als letzter Ausweg nur die Entfernung der sich innerhalb des Auges befindlichen Linse und die Implantation einer künstlichen Intraokularlinse, kurz IOL genannt.

Wo in der Vergangenheit nur sphärisch geformte IOLs eingesetzt wurden, erlaubt der medizinische Fortschritt mittlerweile auch den Einsatz von asphärisch geformten Linsen, deren Hauptzweck darin liegt, die Abbildungsfehler einer ungleichmäßig verkrümmten Hornhaut zu kompensieren. Eine besonders häufig auftretende Art der Hornhautverkrümmung stellt der Astigmatismus dar. Bei dieser Fehlsichtigkeit wird das in das Auge tretende Licht nicht gleichmäßig gebrochen und auf einen Punkt fokussiert. Die Kornea oder Hornhaut weist beim Astigmatismus keine konstante Krümmung auf, sondern kann in guter Näherung durch eine Torusoberfläche angenähert werden, die durch zwei Krümmungswerte entlang zweier zueinander senkrechter Oberflächenrichtungen charakterisiert werden kann. In der einen Richtung weist die Hornhaut die geringste Krümmung auf (flache Hornhautachse), wohingegen die Korneakrümmung senkrecht dazu maximal ist (steile Hornhautachse). Für eine vollständige Charakterisierung des Astigmatismus muss neben den zwei erwähnten Krümmungswerten noch eine Angabe über die Orientierung der Richtung mit der beispielsweise größten Krümmung, die sogenannte Achslage, erfolgen.

Zur Bestimmung der Oberflächenkrümmung sowie z.B. der Astigmatismusachse des Auges kommen diverse Diagnosegeräte zum Einsatz. Diese basieren mit wenigen Ausnahmen auf der Auswertung von Augenbildern, in denen der Abstände zwischen den entweder punktförmigen oder ringartigen Reflexen, welche durch eine entsprechende Beleuchtungsanordnung auf der Hornhautoberfläche entstehen, analysiert werden. So erscheinen die Reflexe einer beispielsweise ebenen, kreisrunden Anordnung von Leuchtdioden bei einem astigmatischen Auge im Kamerabild ellipsenähnlich angeordnet.

Eine Intraokularlinse, die diese Fehlbildung der Kornea auszugleichen sucht, muss selbst in geometrisch ähnlicher Weise geformt sein. Zu diesem Zweck werden torische Linsen verwendet, welche sich gerade dadurch auszeichnen, dass sie in zwei zueinander senkrechten Richtungen eine maximale bzw. minimale Krümmung aufweisen. Die Achse einer torischen Intraokularlinse wird durch auf die Linsenoberfläche aufgebrachte Markierungen gekennzeichnet. Meist bestehen diese Markierungen aus Punkten oder Strichen, deren gedachte Verbindungslinie die Linsenachse definiert.

Für eine optimale Korrektur des Hornhautastigmatismus muss die torische Intraokularlinse beim Einsetzen in das Auge exakt ausgerichtet werden, d.h. sie muss während der Operation durch den Arzt so gedreht werden, dass deren Achse möglichst gut mit der Astigmatismusachse der Hornhaut, welche während der Diagnose zu einem früheren Zeitpunkt bestimmt wurde, übereinstimmt. Das Problem hierbei ist jedoch, dass die relative torsionale Orientierung des Auges zwischen der Diagnosemessung und zum Zeitpunkt der OP im Allgemeinen nicht als konstant und bekannt vorausgesetzt werden kann.

Die Druckschrift WO 2010/009897 A1 beschreibt ein Augenchirurgiesystem und entsprechendes Verfahren, bei dem ein präoperatives erstes Bild eines zu operierenden Auges in einem Bildspeicher gespeichert wird. Eine Kamera dient zur intraoperativen Aufnahme eines zweiten Bildes des zu operierenden Auges. Eine Bildverarbeitungsvorrichtung ermittelt aus dem ersten Bild und dem zweiten Bild einen Orientierungswert. Eine Anzeigevorrichtung erzeugt eine Darstellung einer Markierung in Abhängigkeit von dem Orientierungswert.

Die Druckschrift WO 2010/046371 A1 beschreibt ein Bildverarbeitungsverfahren für computerunterstützte Augenoperationen. Dabei wird ein Referenzbild des Auges aufgenommen und mit Kontextinformationen angereichert. Das Referenzbild wird mit einem Echtzeitbild des Auges registriert, und die Kontextinformation wird dem Echtzeitbild so überlagert, dass sie an derselben Position unabhängig von einer Augenbewegung wiedergegeben wird.

DE 10 2009 053 208 A1 beschreibt eine Vorrichtung für Augenoperationen mit einer Bilderkennungsvorrichtung, die den Operationsvorgang beobachtet und qualitativ analysiert, um dem Chirurgen eine Rückmeldung zu geben. Die Rückmeldung kann das Führen eines Operationsinstruments oder das laterale oder rotative Verschieben bzw. Verdrehen einer Intraokularlinse zum Gegenstand haben.

Die Druckschrift WO 2015/176699 A2 offenbart eine Vorrichtung zur Verfolgung und Kompensation von Augenbewegungen bei Laserbehandlungen des Auges. Dabei wird ein Merkmal des Auges sowohl mit Umgebungslicht als auch mit einem Tracking Licht beleuchtet. Das Bild des Merkmals des Auges wird erfasst und daraus werden Signale erzeugt, die der Bewegung des Bildes entsprechen. Filter trennen die Signale, so dass nur die Signale einer ersten Komponente aufgrund des Tracking Lichts zur Verfolgung des Auges verwendet werden. Aufgrund des Tracking Signals wird ein Muster von Laserschusspunkten auf das Auge gesteuert, um die optische Achse des Laserstrahls mit der Sichtachse des Auges im Wesentlichen zentriert zu halten

Die Druckschrift WO 2009/135084 A1 offenbart ein System zur Erfassung der Augenbewegung für Augenlaseroperationen. Dabei wird das Auge mit einer Lichtquelle 11 mit einem ersten, polarisierten Licht beleuchtet. Eine Bilderfassungseinrichtung oder Kamera erzeugt aus dem vom Auge reflektierten Licht ein Bild des Auges. Aus dem reflektierten Licht wird ein zweites Licht herausgefiltert, das den Polarisierungszustand hat. Eine Recheneinheit bestimmt die Augenbewegung aus dem Bild des Auges. Durch die Beleuchtung mit polarisiertem Licht und die Bestimmung der Augenbewegung aus diffusem reflektierten Licht werden störende Reflektionen unterdrückt

Weitere Druckschriften auf diesem Gebiet sind zum Beispiel US 8414123 B2, EP 2184005 B1, WO 2012041349 A1 und DE102008034490 A1.

Im Stand der Technik erfolgt eine Registrierung als integraler Bestandteil, d.h. es wird ein präoperatives Bild mit einem operativen verglichen, um daraus die Torsion zu bestimmen.

Die korrekte Orientierung spielt jedoch nicht nur bei torischen Linsen und astigmatischen Augen eine entscheidende Rolle. Vielmehr ist sie immer dann von Bedeutung, wenn die Intraokularlinse bezüglich Geometrie oder anderer Eigenschaften, beispielsweise optischer Eigenschaften, keine Zylindersymmetrie aufweist. So sind auch asphärische Intraokularlinsen denkbar, die keine torische Form haben und beispielsweise einen patientenspezifischen Keratokonus auszugleichen versuchen, oder auch Multifokallinsen mit einer ebenfalls patientenspezifischen und nicht unbedingt zylindersymmetrischen Anordnung der verschiedenen Zonen. Auch bei diesen Geometrien käme es auf eine torsional lagerichtige Ausrichtung der Linsen an.

Ähnliche Probleme treten auch bei anderen Typen von refraktiven Operation auf. Als Beispiele seien hier Laserbehandlungen des Auges wie z.B. sogenannte LASIK-Operationen oder PRK-Operationen genannt. Bei diesen Operationen wird Hornhautgewebe mittels Laserbeschuss definiert abgetragen, um Abbildungsfehler des Auges zu korrigieren. Die Abtragmuster basieren auf einer präoperativen Diagnose und müssen durchaus nicht zylindersymmetrisch sein. Bei astigmatischen Augen ist das Abtragmuster beispielsweise nur symmetrisch zur Astigmatismusachse. Somit ist auch hier die Kenntnis der Augentorsion zwischen Diagnose und der aktuellen Orientierung bei der Operation dringend für eine Drehung der Schusskoordinatenliste des Lasers erforderlich.

Die relative Verdrehung des Auges um die Blickachse zwischen dem Diagnosezeitpunkt und unmittelbar vor dem operativen Eingriffs wird häufig als statische Torsion bezeichnet. Diese tritt, wenn keine weiteren Maßnahmen ergriffen werden, mit hoher Wahrscheinlichkeit immer auf. Hierfür können mehrere Faktoren verantwortlich sein.

Einerseits können die während der Diagnose und der Operation verwendeten Kameras relativ zum Kopf des Patienten gegeneinander verdreht sein oder aber die Kopfhaltung relativ zur Kamera kann sich bei der Operation gegenüber der Haltung bei der Diagnose verändert haben. Andererseits kann es tatsächlich zu einer torsionalen Augenbewegung beim Übergang von einer sitzenden Position während der Diagnose zu einer liegenden Position des Patienten während der Operation kommen.

Neben der statischen Torsion kann intraoperativ zusätzlich eine sog. dynamische Torsion auftreten. Diese hat ihre Ursache in akuten torsionalen Augenbewegungen, in weiteren Kopfbewegungen des Patienten oder ist möglicherweise durch die Verwendung von Operationsinstrumenten durch den Arzt induziert.

Gegenwärtig werden hauptsächlich drei automatische oder halbautomatische Verfahren zur Vermeidung von torsionsbedingten Ausrichtungsfehlern beim Einsetzen einer Intraokularlinse angewendet. Bei einem sehr häufig zum Einsatz kommenden Verfahren wird das Patientenauge mit einem Stempel und wasserfester Tinte markiert. Die während der Diagnose ermittelte Orientierung der Astigmatismusachse des Auges wird dann relativ zu dieser Markierung angegeben. Während der Operation können diese Marker z.B. mittels Bildverarbeitungsalgorithmen detektiert werden.

Sogenannte Surgery Guidance Systeme erkennen die Position der Markierungen und blenden z.B. die Sollposition der Linsenachse relativ zu den detektierten Markierungen in den Strahlengang des Mikroskops, und somit sichtbar für den Arzt, ein.

Neuere bekannte Verfahren benötigen theoretisch keine Markierungen. Beispielsweise können sichtbare anatomische Eigenschaften des Auges zur Ermittlung der Augentorsion zwischen Diagnose und Operation herangezogen werden. Hier bieten sich die Blutgefäße der Sklera oder Bindehaut bzw. das Irismuster an.

Nachteilig bei diesen Verfahren ist die Tatsache, dass während der Operation sehr häufig keine Irismuster sichtbar sind, da die Pupille meist medikamentös erweitert wird und andererseits zu wenige oder nur nicht markante Blutgefäße vorhanden oder durch die Lider bei der Diagnose verdeckt sein können.

Häufig entstehen auch bei der Lensektomie (Linsenentfernung), im Speziellen bei der Linsenfragmentation mittels Femtosekundenlaser, bedingt durch einen mechanischen Ansaug- und Fixierprozess des Auges, sklerale Hämatome, die die skleralen Gefäße derart überlagern, dass eine Registrierung des Diagnosebildes mit einem Augenbild aus der Operation nach der Entfernung der natürlichen Linse erschwert oder sogar unmöglich gemacht wird.

Andere bekannte Verfahren und Systeme basieren auf der Auswertung von Lichtwellenfronten, welche im Ergebnis eine Aussage über die optischen Eigenschaften des Auges erlauben. Nach der Entfernung der natürlichen Linse verbleibt die Kornea als einzige brechende Fläche. Eine Messung zu diesem Zeitpunkt ermittelt die Abbildungsfehler allein der Kornea und erlaubt z.B. eine Angabe über die Orientierung der Astigmatismusachse. Besonders nachteilig bei derartigen Systemen ist die Abhängigkeit der Ergebnisse von allen die Lichtbrechung beeinflussenden Faktoren wie beispielsweise mechanische Deformationen der Hornhaut durch Operationsinstrumente, Gasbläschen in z.B. der Augenvorderkammer oder der Einsatz von Viskoelastika.

Neben den beschriebenen automatischen bzw. halbautomatischen Verfahren zur Vermeidung von IOL-Ausrichtungsfehlern existiert noch eine Reihe von weiteren halbautomatischen bzw. manuellen Verfahren, auf die nicht näher eingegangen wird. Hier wird auf die eine oder andere Weise die Torsion vom Arzt mitbestimmt, z.B. durch das manuelle Übereinanderschieben von polartransformierten Bildern, die jeweils die ringartige Bildregion jenseits des Limbus während der Diagnose und während der Operation darstellen.

Gegenwärtig wird bei Kataraktoperationen die Sollorientierung der Linsenachse der einzusetzenden Intraokularlinse in den Strahlengang des Operationsmikroskops optisch eingeblendet und erscheint dem Arzt in die Bildszene eingebettet. Alternativ existiert die Möglichkeit, auf einem externen Sichtgerät die von einer Kamera beobachtete Operationsszene inklusive der Sollposition der IOL-Achse online darzustellen. Der Arzt kann mit Hilfe dieser Anzeigen die eingesetzte Intraokularlinse so lange drehen, bis die Markierungen auf der Linse mit der eingeblendeten Sollachse zusammenfallen. Neben der bereits erwähnten Torsion treten naturbedingt auch nichttorsionale Kopf- oder Augenbewegungen auf, welche durch Verfolgungsalgorithmen detektiert werden können. Damit lässt sich eine mit dem Auge mitwandernde Darstellung der Sollachse sowie weiterer, relevanter Informationen realisieren.

Bei Operationstypen, bei denen die natürliche Linse im Auge verbleibt und die Korrektur der Sehfehler über eine Neuformung der Hornhaut mittels Laserablation erzielt werden soll, wird die Torsion in ähnlicher Weise indirekt bestimmt, d.h. eine Bildverarbeitung ermittelt die relative Verdrehung eines aktuellen Operationsbildes gegenüber einem Referenzbild aus der Diagnose. Dieser Prozess kann mehrstufig sein, in dem Sinne, dass die Bildverarbeitung zunächst die Torsion zwischen einem Diagnosebild und einem Operationsbild berechnet, wobei das Operationsbild das Auge noch vor dem tatsächlichen Eingriff zeigt, und anschließend dieses Operationsbild als neue Referenz festgelegt wird, zu der alle folgenden Operationsbilder bei der Berechnung der dynamischen Torsion in Beziehung gesetzt werden.

Im Gegensatz zu Kataraktoperationen, bei denen die Pupille medikamentös erweitert wird, kann man zur Torsionsermittlung bei refraktiven Korneaoperationen meist auch auf das Irismuster zurückgreifen. Hierbei gibt es allerdings das Problem, dass bei der Diagnose Geräte zum Einsatz kommen können, die zur Ermittlung der Topographie der Kornea eine Vielzahl von beispielsweise hellen Ringen auf der Augenoberfläche produzieren und damit Irisstrukturen überdecken können, die dadurch für die Algorithmen unbrauchbar werden. Dies wirkt sich nachteilig auf die Berechnung der Torsion aus oder macht sie unter Umständen auch zu ungenau oder unmöglich.

Wollte man Blutgefäße als Merkmale für eine Registrierung heranziehen, so ergibt sich das Problem, dass diese zwar meist gut während der Diagnose sichtbar sind aber während der Operation im Allgemeinen von der Kamera nicht wahrgenommen werden können, da IR-Licht zur Beleuchtung verwendet wird. Somit bleibt momentan nur die Iris als Merkmalsträger bei der Berechnung der Torsion übrig.

Insbesondere intraoperativ ergibt sich bei der Berechnung der Torsion zusätzlich die Schwierigkeit, dass die Iris und die darin enthaltenen Merkmale mit fortschreitender Behandlung immer undeutlicher werden. Dies liegt darin begründet, dass die korneale Oberfläche beim Laserabtrag immer rauer wird. Die Transparenz nimmt dadurch ab und die Irismerkmale können unter Umständen nicht mehr zur Berechnung der Torsion herangezogen werden. Zu einem ähnlichen Effekt kommt es beim sogenannten Flapschnitt bei LASIK-Operationen. Die Schnittfläche weist im Allgemeinen eine Rauheit auf, die sich Nachteilig auf die Detektion der Irismuster auswirkt, unabhängig davon, ob der Schnitt mit einem Mikrokeratom oder mittels Femtosekundenlaser (Femto-LASIK) durchgeführt wurde.

Es ist die Aufgabe der Erfindung, die vorgenannten Nachteile der bekannten Systeme und Verfahren zu überwinden und die Genauigkeit bei chirurgischen Eingriffen in das Auge zu erhöhen. Insbesondere soll die Genauigkeit beim Einsatz von Intraokularlinsen und bei der Durchführung von Laserablationen erhöht und die resultierende Sehstärke nach dem Eingriff verbessert werden.

Diese Aufgabe wird gelöst durch die Vorrichtung gemäß Patentanspruch 1, das Operationsmikroskop gemäß Patentanspruch 9, das Computerprogramm gemäß Patentanspruch 10 und die Steuereinheit gemäß Patentanspruch 20. Weitere vorteilhafte Merkmale ergeben sich aus den abhängigen Ansprüchen, der Beschreibung und den Zeichnungen.

Vorteile und Eigenschaften, die im Zusammenhang mit den Merkmalen der erfindungsgemäßen Vorrichtung genannt sind, gelten entsprechend auch für die Merkmale des erfindungsgemäßen Computerprogramms und umgekehrt.

Die erfindungsgemäße Vorrichtung zur Bestimmung der Ausrichtung des Auges bei Augenoperationen ist in Patentanspruch 1 definiert und umfasst eine Beleuchtungseinrichtung, eine Kameraeinrichtung, um ein Bild während der Augenoperation zu erfassen, und eine Recheneinheit zur Bestimmung der Ausrichtung des Auges aus dem erfassten Bild, wobei die Beleuchtungseinrichtung eine Lichtquelle oder Lichtquellenanordnung umfasst, deren Reflexion an der Kornea des Auges in dem Bild der Kameraeinrichtung als ein Lichtmuster detektierbar ist, und die Recheneinheit aus dem im Bild der Kameraeinrichtung erfassten Lichtmuster mindestens eine Eigenschaft des Auges ermittelt, die von der momentanen Ausrichtung des Auges abhängig ist.

Das erfindungsgemäße Computerprogramm zur Steuerung eines Augenchirurgiesystems ist in Patentanspruch 10 definiert und ist zur Durchführung der folgenden Schritte ausgestaltet: Beleuchten eines Auges und Erfassen eines Bildes des Auges; Bestimmung der momentanen Ausrichtung des Auges aus dem erfassten Bild, wobei die Gestalt einer speziell gestalteten Lichtquelle oder Lichtquellenanordnung nach Reflexion an der Kornea des Auges als ein Lichtmuster erfasst wird, und aus dem erfassten Lichtmuster mindestens eine Eigenschaft des Auges bestimmt wird, die die momentane Ausrichtung des Auges kennzeichnet.

Weiterhin wird ein Verfahren zur Bestimmung der Ausrichtung des Auges beschrieben mit den Schritten: Erzeugen eines Lichtmusters auf dem Auge und Erfassen des Lichtmusters während sich der Patient in der Operationslage befindet; und Bestimmen mindestens einer Eigenschaft des Auges aus dem in der Operationslage des Patienten erfassten Lichtmuster, die die momentane Ausrichtung des Auges kennzeichnet.

Dadurch, dass die relevanten Eigenschaften bzw. Parameter des Auges direkt während der Operation bestimmt werden, sind diese vom Bildmaterial aus der Diagnose unabhängig.

Insbesondere wirkt sich eine zwischen dem Diagnosezeitpunkt und dem Zeitpunkt des Eingriffs erfolgte Torsion des Auges nicht mehr schädlich auf das Operationsergebnis aus. Die Bestimmung der Torsion erfolgt intraoperativ, das heißt, wenn sich der Patient bereits in der Operationslage befindet, was auch den Zeitraum während der Operation umfasst. Die torsionale Lage des Auges zum Diagnosezeitpunkt spielt keine Rolle mehr. Stattdessen ist nur die momentane Ausrichtung des Auges während der Operation von Bedeutung.

Durch diesen Ansatz werden die im Stand der Technik bestehenden Probleme und Hindernisse gelöst, denn man ist weder von der Existenz von in Diagnose- und Operationsbild gleichzeitig sichtbaren Blutgefäßen und Irisstrukturen abhängig, noch stellen die durch die Fixierung des Auges während der Linsenfragmentation mittels Femtosekundenlaser während der Lensektomie induzierten skleralen Hämatome ein Problem bei der Registrierung zwischen Diagnose- und Operationsbild dar. Insbesondere kann z.B. bei Kataraktoperationen auf die Ermittlung der torsionalen Differenz zum Diagnosezeitpunkt verzichtet werden.

Bevorzugt wird eine aus dem Lichtmuster bestimmte Eigenschaft zur Steuerung einer Lichtmarkierung auf dem Auge in Abhängigkeit von der momentanen Ausrichtung des Auges verwendet. Dadurch erhält der Arzt eine wichtige Hilfestellung für die Operation. Sie kann aber auch bei einer Laserbehandlung zur Korrektur der Position der Ablationsorte in Abhängigkeit von der momentanen Ausrichtung des Auges verwendet werden. Dadurch wird die Genauigkeit von Laserbehandlungen erhöht.

Die ermittelte Eigenschaft des Auges kann zum Beispiel ein ein- oder mehrdimensionaler Parameter sein. Die Eigenschaft bzw. der Parameter ist beispielsweise geometrischer, differentialgeometrischer, topographischer oder optischer Natur.

Vorteilhaft ist die aus dem Lichtmuster ermittelte Eigenschaft eine spezifische topografische Eigenschaft des Auges. Sie kann zum Beispiel die Irisoberfläche, die Skleraoberfläche oder auch die Korneaoberfläche kennzeichnen.

Insbesondere kennzeichnet die aus dem Lichtmuster ermittelte Eigenschaft bzw. der Parameter die Lage der Astigmatismusachse des Auges und/oder eine hierzu in definierter Beziehung stehende Richtung. Dadurch können z.B. Intraokularlinsen exakt lagerichtig eingesetzt werden, ohne dass eine zwischen dem Diagnosezeitpunkt und dem Zeitpunkt des Einsatzes der Intraokularlinse erfolgte Torsion des Auges eine Rolle spielt.

Die aus dem Lichtmuster ermittelte Eigenschaft wird bevorzugt durch Erhebung von Daten des Auges während der Operation bestimmt, wobei die Daten insbesondere aus topometrischen, topografischen, keratometrischen, keratografischen oder ophthalmometrischen Messungen gewonnen werden.

Bevorzugt werden die momentanen Daten oder daraus ableitbare Größen mit entsprechenden während der Diagnose ermittelten Daten oder daraus ableitbaren Größen verglichen, um daraus eine Lageveränderung während der Operation gegenüber dem Diagnosezeitpunkt zu bestimmen. Als Lageveränderung kann insbesondere eine momentane Torsion des Auges bestimmt werden.

Dadurch kann insbesondere eine torsionale Beziehung zu äquivalenten Parametern des Auges zum Diagnosezeitpunkt ermittelt werden. Das heißt, durch Lagevergleich der momentanen Augendaten und daraus ableitbarer Parameter oder Eigenschaften mit entsprechenden Daten oder daraus ableitbarer Parameter aus der Diagnose, die z.B. die Astigmatismusachse kennzeichnen, kann die aktuelle Torsion gegenüber der Torsion zum Diagnosezeitpunkt bestimmt werden. Damit kann bei Bedarf auch eine refraktive Laserbehandlung exakter gesteuert werden.

Die während der Operation erhobenen Daten und die zugehörigen Kamerabilder werden beispielsweise gespeichert und als Referenzdaten oder - bilder für nachfolgende Schritte zur Berechnung der intraoperativen Lageveränderung, insbesondere der Torsion, verwendet.

Beispielsweise erfolgt eine wiederholte oder erneute Ermittlung der relevanten Parameter des Auges. Vorteilhaft werden ein oder mehrere Augenbilder aus der Operation, für die die oben erwähnten Daten erhoben werden und die z.B. die Astigmatismusachse kennzeichnen, gespeichert und als Referenzbilder für den folgenden Schritt verwendet. In dem nachfolgenden Schritt wird dann die intraoperative Torsion zwischen einem aktuellen Operationsbild, das das Auge zu einem Zeitpunkt nach dem Speichern eines dieser Referenzbilder zeigt, und einem dieser Referenzbilder mit Mitteln der Bildverarbeitung bestimmt.

Dadurch kann beispielsweise bei refraktiven Operationen wie zum Beispiel LASIK oder PRK, die Schusspositionen des Lasers bei Torsionen des Auges während der Operation entsprechend nachgeführt werden.

Insbesondere wird gemäß der Erfindung die Behandlung bei einer refraktiven Laseroperation aufgrund der momentan ermittelten Eigenschaft des Auges angepasst oder korrigiert. Dies kann beispielsweise durch die Ausrichtung des Schussmusters des Ablationslasers aufgrund der momentan ermittelten geometrischen Parameter des Auges während der Laserbehandlung erfolgen.

Die aufgrund der geometrischen oder topografischen Eigenschaft auf dem Auge erzeugte Lichtmarkierung zeigt vorteilhafterweise während der Augenoperation auf dem Auge die momentane Ausrichtung des Auges, insbesondere in Bezug auf den Drehwinkel um die Achse der Blickrichtung, und/oder eine für die Operation benötigte Ortsmarkierung an. Die Lichtmarkierung zeigt zum Beispiel während der Augenoperation auf dem Auge die Soll-Ausrichtung einer in das Auge einzusetzenden Intraokularlinse und/oder die Soll-Position eines durchzuführenden Schnittes bzw. einer Inzision an.

Die Lichtmarkierung zeigt insbesondere während der Augenoperation die Richtung der Astigmatismusachse des Auges auf dem Auge an. Optional kann auch eine Achse angezeigt werden, die in einer definierten Beziehung zur Astigmatismusachse steht. Dadurch kann der Arzt zum Beispiel eine Intraokularlinse beim Einsetzen exakt anhand der Astigmatismusachse oder zu dieser in Beziehung stehender Achsen ausrichten.

Durch diese Merkmale werden dem Arzt Informationen zur Verfügung gestellt, welche ihm zum Beispiel eine korrekte Ausrichtung einer in Form und Eigenschaft nicht zylindersymmetrischen Linse oder auch die exakte Platzierung eines Schnittes erlauben. Dadurch wird ihm eine wichtige Unterstützung oder Hilfestellung gegeben. Die Angabe der Astigmatismusachse des Auges oder einer zu dieser in definierter Beziehung stehender Achse kann zum Beispiel im aktuellen Operationsbild beim Einsetzen einer torischen Intraokularlinse erfolgen.

Vorteilhaft wird das Lichtmuster auf dem Auge mit einer diffusen oder afokalen Beleuchtungsquelle erzeugt. Eine diffuse oder afokale Beleuchtung bietet insbesondere zwei wesentliche Vorteile. Einerseits lassen sich auf eine einfache Weise sehr spezifische Reflexformen realisieren und andererseits entfällt die Notwendigkeit einer exakten Ausrichtung, wie sie bei einer fokussierten Beleuchtung notwendig ist. Die Ausrichtung der Beleuchtung an die Entfernung des Auges vom Mikroskop und an die Entfernung der Beleuchtungseinrichtung zum Auge muss nicht angepasst werden um Reflexe zu erzeugen, die von der Kamera detektierbar sind. Zur Erzeugung einer afokalen Beleuchtung können z.B. streuende Folien oder Glasscheiben als Vorsatz für beispielsweise LEDs oder Lichtleiter verwendet werden.

Die diffusen oder afokalen Lichtquellen sollen Reflexe erzeugen, die von der Kamera beobachtet werden können. Aus der Lage der Reflexe kann man z.B. auf die Astigmatismusachse schließen. Der diffuse Charakter dieser Lichtquellen ermöglicht eine Formgebung und ist extrem tolerant gegenüber einer variierenden Entfernung zum Auge und gegenüber der Entfernung von Auge zu Kamera.

Afokal bedeutet, dass es für das emittierte Licht keine Vorzugsrichtung gibt wie beispielsweise bei Benutzung einer LED der Fall wäre. Diese bündelt im Allgemeinen das Licht durch die Form Ihres Gehäuses. Ist diese Bündelung zu ausgeprägt, dann kann es sein, dass die Reflexe von der Kamera unter bestimmtem Bedingungen (ungünstige Abstrahlrichtung bei bestimmten Entfernungen der LED vom Auge und des Auges von der Kamera) entweder nicht mehr wahrgenommen werden oder aber der Kamera nicht mehr gleichmäßig hell erscheinen. Letzteres ist aber für die Bildverarbeitung wichtig, die beispielsweise über Schwellwertoperationen die Position der Reflexe im Bild ermitteln muss.

Ein weiterer Vorteil von diffusen Lichtquellen ist die Fähigkeit zur speziellen Formgebung von Reflexen. Mit einer gebündelten, also fokalen, Beleuchtung ist es nicht möglich, eine spezifisch geformte Lichtquelle zu erzeugen (Kreis, Rhombus, Dreieck etc.), deren spezielle Gestalt nach der Reflexion an der hochreflektiven Kornea als solche auch von einer Kamera wahrgenommen werden kann.

Das auf dem Auge erzeugte Lichtmuster umfasst spezifische Reflexformen. Der Vorteil von spezifischen Reflexformen liegt insbesondere darin, dass Fremdreflexe von den Algorithmen der Bildverarbeitung identifiziert und eliminiert werden können. Dadurch wird die Ermittlung der Astigmatismusachse erleichtert oder sogar erst ermöglicht. Unterstützt würde dieses Vorgehen noch dadurch, dass die einzelnen Lichtquellen optional Licht verschiedener Wellenlänge emittieren oder gepulst betrieben werden.

Neben der Astigmatismusachse kann z.B. auch die Krümmung der Kornea abgeleitet werden. Allgemein kann man von geometrischen oder topographischen Parametern sprechen.

Bevorzugt fällt das Licht zur Erzeugung der Lichtmarkierung auf dem Auge unter einem Winkel α zur Achse einer erfassenden Kamera ein.

Dies wird zum Beispiel dadurch erreicht, dass der Projektor zur Erzeugung der Lichtmarkierung nicht im Mikroskop integriert ist, sondern sich außerhalb des Mikroskopgehäuses befindet bzw. daran befestigt ist. Dadurch schließen die Achse der integrierten Mikroskopkamera und die Einfallsrichtung der Lichtmarkierung einen nichtverschwindenden Winkel ein. Diese Tatsache kann dazu benutzt werden, die Augenausrichtung zu bestimmen.

Der Projektor oder Mustergenerator soll z.B. eine Linie oder ein Linienpaar auf das Auge projizieren. Der Winkel, unter dem das Muster gegenüber der Kameraachse auf dem Auge generiert wird, liegt bevorzugt im Bereich von 5 Grad bis 85 Grad, insbesondere bevorzugt zwischen 5 Grad und 50 Grad. Wichtig ist hierbei, dass dieser Winkel größer als Null ist, damit zum Beispiel über Triangulationsalgorithmen die Lage z.B. der Limbusebene und damit die Blickrichtung berechnen werden kann. Dadurch können Entfemungsinformationen aus den Bildkoordinaten des projizierten Musters extrahiert werden. Dies ist ein großer Vorteil gegenüber beispielsweise einer koaxialen Erzeugung und Erfassung der Lichtmarkierung, bei der die Projektionsrichtung des Musters und die Kameraachse keinen Winkel miteinander einschließen.

Mit dem Muster kann also dem Arzt einerseits die torsionale Orientierung mitgeteilt werden, die die IOL haben soll, und andererseits kann man auch zusätzlich die Blickrichtung des Patientenauges bestimmen. Zu diesem Zweck ist das projizierte Muster komplexer als eine Linie, z.B. ein Linienpaar. Durch drei oder mehr Schnittpunkte des Musters mit dem Limbus ergibt sich eine Ebene im Raum. Mit nur einer Linie kann man zum Beispiel die Entfernung der Pupillenmitte ermitteln.

Die Vorrichtung zur Bestimmung der Ausrichtung des Auges während einer Augenoperation umfasst insbesondere eine Beleuchtungseinrichtung zur Erzeugung eines Lichtmusters auf dem Auge eines sich in der Operationslage befindlichen Patienten; und eine Kameraeinrichtung, um das auf dem Auge erzeugte Lichtmuster während der Augenoperation zu erfassen; sowie eine Recheneinheit, die derart ausgestaltet ist, dass sie während der Augenoperation aus dem erfassten Lichtmuster mindestens eine Eigenschaft des Auges ermittelt, die von der momentanen Ausrichtung des Auges abhängig ist.

Die oben in Bezug auf das Verfahren beschriebenen Vorteile gelten entsprechend auch für die Merkmale der erfindungsgemäßen Vorrichtung.

Die ermittelte Eigenschaft oder der Parameter ist beispielsweise eine geometrische, differentialgeometrische, topografische, keratografische oder optische Eigenschaft des Auges, die die momentane Ausrichtung des Auges kennzeichnet.

Vorteilhaft ist eine Einrichtung zur Befestigung an einem Operationsmikroskop oder zum Einschwenken der Beleuchtungseinrichtung vorgesehen. Dadurch und durch die folgenden Merkmale werden die weiter oben im Zusammenhang mit dem erfindungsgemäßen Verfahren beschriebenen Vorteile ermöglicht.

Vorteilhafterweise ist eine Steuereinheit zur Ansteuerung einer zweiten Beleuchtungseinrichtung zur Erzeugung einer Lichtmarkierung auf dem Auge aufgrund des momentan ermittelten geometrischen Parameters während der Augenoperation vorgesehen. Die Steuereinheit kann jedoch zum Beispiel auch einen Ablationslaser aufgrund des momentan während der Augenoperation ermittelten geometrischen Parameters ansteuern. Dabei wird nur die Information bzgl. der statischen Torsion an den Behandlungslaser übermittelt. Dieser dreht daraufhin seine Schussliste bzw. ändert die Schusspositionen.

Die aus dem Lichtmuster ermittelte Eigenschaft oder Parameter kennzeichnet zum Beispiel eine spezifische lageabhängige Charakteristik des Auges. Diese Eigenschaft ist insbesondere eine spezifische geometrische, differentialgeometrische, topografische, keratografische oder optische Eigenschaft.

Vorteilhaft kennzeichnet die aus dem Lichtmuster ermittelte Eigenschaft oder Parameter die Lage der Astigmatismusachse des Auges oder eine hierzu in definierter Beziehung stehende Richtung.

Bevorzugt ist die Beleuchtungseinrichtung zur Erzeugung des Lichtmusters derart ausgestaltet, dass sie diffuses oder afokales Licht erzeugt.

Insbesondere ist eine zweite Beleuchtungseinrichtung zur Erzeugung einer Lichtmarkierung auf dem Auge vorgesehen, die während der Augenoperation auf dem Auge Informationen wie z.B. die momentane Ausrichtung eines Augenparameters und/oder eine für die Operation benötigte Ortsmarkierung anzeigt.

Vorteilhaft zeigt die Lichtmarkierung die Soll-Ausrichtung einer in das Auge einzusetzenden Intraokularlinse und/oder die Soll-Position einer durchzuführenden Inzision auf dem Auge an.

Die Lichtmarkierung kann insbesondere die Astigmatismusachse des Auges oder eine zu dieser in definierter Beziehung stehender Achse auf dem Auge anzeigen.

Vorteilhafterweise korrigiert die Steuereinheit aufgrund der gewonnenen Informationen über die erfolgte Torsion die Ausrichtung des Schussmusters des Ablationslasers aufgrund der momentan ermittelten geometrischen Parameter des Auges während der Laserbehandlung.

Insbesondere ist die Beleuchtungseinrichtung zur Erzeugung der Lichtmarkierung derart angeordnet, dass der Einfall unter einem Winkel α zur Achse einer erfassenden Kamera erfolgt.

Vorteilhafterweise ist die Beleuchtungseinrichtung derart ausgestaltet ist, dass auf dem Auge spezifische Reflexformen erzeugt werden.

Gemäß einem Aspekt der Erfindung wird ein Operationsmikroskop für die Augenchirurgie geschaffen, das eine erfindungsgemäße Vorrichtung umfasst.

Gemäß einem weiteren Aspekt wird eine Steuereinheit für Augenchirurgiesysteme geschaffen, die zur Durchführung des Verfahrens ausgestaltet ist.

Die Steuereinheit umfasst insbesondere eine Recheneinheit, die aus einem während der Augenoperation auf dem Auge erfassten Lichtmuster mindestens eine Eigenschaft oder Parameter des Auges ermittelt, der die momentane Ausrichtung des Auges kennzeichnet, wobei die Steuereinheit derart ausgestaltet ist, dass sie eine optische Anzeigevorrichtung und/oder einen Ablationslaser steuert.

Weiterhin wird ein Computerprogramm zur Steuerung eines Augenchirurgiesystems geschaffen, das Programmschritte zur Durchführung des Verfahrens umfasst.

Nachfolgend wird die Erfindung anhand der Figuren beispielhaft beschrieben. Es zeigen:
- **Figur 1**: eine Vorrichtung gemäß einer bevorzugten Ausführungsform der Erfindung, die an einem Operationsmikroskop befestigt ist;
- **Figuren 2 a-c**: bevorzugte Beispiele für auf dem Auge erzeugte Lichtmuster;
- **Figuren 3a und 3b**: verschiedenartige Lichtquellenanordnungen als Beleuchtungseinrichtung zur Erzeugung von Lichtmustern auf dem Auge;
- **Figur 3c**: eine diffuse Lichtquelle der Beleuchtungseinrichtung mit einem Diffusorelement;
- **Figuren 3d und 3e**: zeigen Beleuchtungseinrichtungen zur Erzeugung spezifischer Reflexformen auf dem Auge;
- **Figur 4a**: Bilder des Auges bei der Diagnose und während der Operation, wobei eine Verdrehung bzw. Torsion des Auges um die Blickachse zwischen den beiden Aufnahmen stattgefunden hat;
- **Figuren 4b und 4c**: Bilder des Auges mit Lichtreflexen, die spezifische Reflexformen aufweisen;
- **Figur 5**: ein Operationsmikroskop, das die erfindungsgemäße Vorrichtung umfasst;
- **Figur 6**: ein erfindungsgemäßes Operationsmikroskop bei dem die interne Kamera des Operationsmikroskops als Kamera zur Erfassung des auf dem Auge erzeugten Lichtmusters verwendet wird;
- **Figur 7**: ein Operationsmikroskop, das die erfindungsgemäße Vorrichtung 10 umfasst, mit Darstellung der Winkel α1 bzw. α2 mit der optischen Achse der internen Kamera zur Erfassung des Bildes des Auges;
- **Figur 8**: ein Bild eines Auges, das in der Darstellung nach rechts verkippt oder rotiert ist; und
- **Figur 9**: ein Bild eines Auges mit einem darauf projizierten Lichtmuster zur Detektion einer Verkippung des Auges.

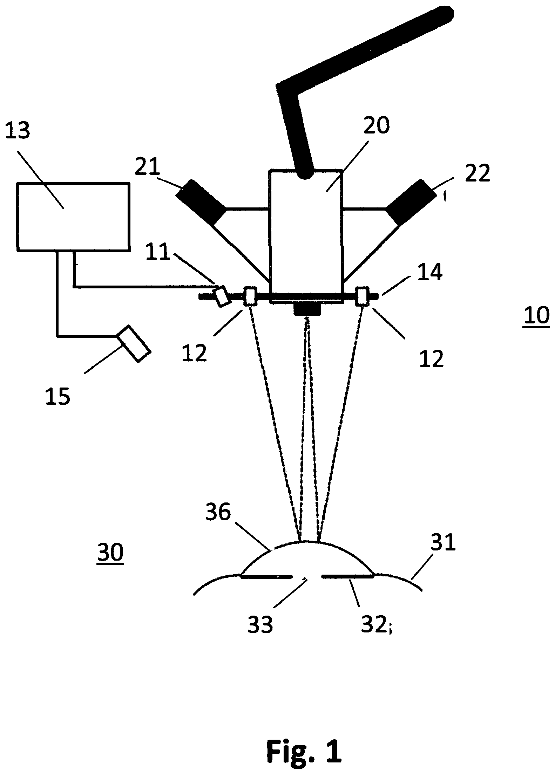

**Figur 1** zeigt eine Vorrichtung 10 gemäß einer bevorzugten Ausführungsform der Erfindung, die an einem Operationsmikroskop 20 befestigt ist. Das Operationsmikroskop 20 umfasst Okulare 21, 22, durch die der Arzt während der Operation auf das Auge 30 des Patienten blickt. An der dem Auge 30 zugewandten Seite des Operationsmikroskops 20 ist die Vorrichtung 10 zur Bestimmung der Ausrichtung des Auges 30 angeordnet. Die Vorrichtung 10 umfasst eine Kamera 11 und eine Beleuchtungseinrichtung 12, die zur Erzeugung eines Lichtmusters auf dem Auge 30 dient. Die Kamera 11 dient zur Erfassung des auf dem Auge erzeugten Lichtmusters und ist mit einer Recheneinheit 13 verbunden. Die Recheneinheit 13 ermittelt aus dem erfassten Lichtmuster einen Parameter, der für die momentane Ausrichtung des Auges 30 charakteristisch ist.

Um mit der Beleuchtungseinrichtung 12 diffuses Licht oder eine afokale Beleuchtung zu erzeugen, werden entsprechend ausgestaltete Lichtquellen verwendet. Es können aber auch zum Beispiel Diffusorelemente als Vorsatz im Strahlengang nach der Lichtquelle angeordnet sein, beispielsweise wenn LEDs oder Lichtleiter als Lichtquellen der Beleuchtungseinrichtung dienen.

Eine Halterung 14 dient als Befestigungseinrichtung, die die Kamera 11 und die Beleuchtungseinrichtung 12 hält und mit dem Operationsmikroskop 20 verbindet. Es kann aber auch eine im Operationsmikroskop integrierte Kamera zur Erfassung des auf dem Auge erzeugten Lichtmusters dienen.

Unter der momentanen Ausrichtung des Auges wird insbesondere die torsionale Lage verstanden, das heißt der Drehwinkel des Auges um die Achse der Blickrichtung.

Optional ist zusätzlich eine zweite Beleuchtungseinrichtung 15 bzw. ein Lichtmustergenerator vorgesehen. Die zweite Beleuchtungseinrichtung 15 dient dazu, eine Lichtmarkierung auf dem Auge 30 zu erzeugen, deren Lage oder Position auf dem Auge 30 aufgrund des von der Recheneinheit 13 aus Bilddaten der Kamera 11 ermittelten Parameters gesteuert wird. Vorteilhaft ist die zweite Beleuchtungseinrichtung 15 ebenfalls an der Halterung 14 befestigt. Mit entsprechenden bewegten oder statischen Mustern lässt sich auch die Blickrichtung bestimmen.

Das Auge 30 umfasst die Sklera 31, die Iris 32, die Pupille 33 und die Kornea 36.

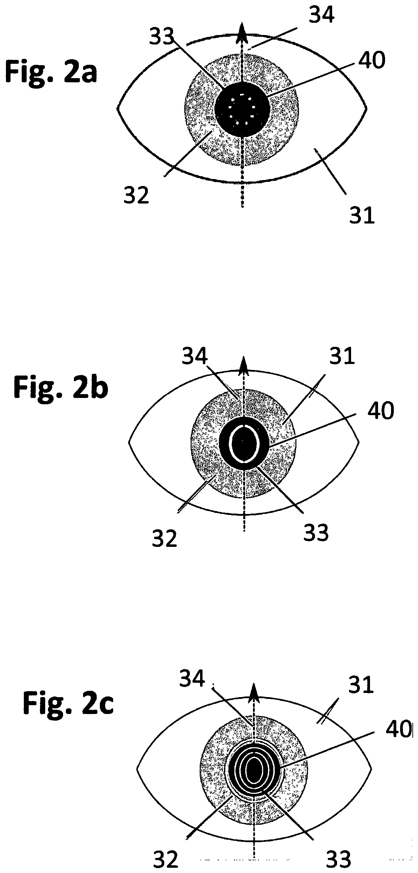

Die **Figuren 2 a-c** zeigen bevorzugte Beispiele für das auf dem Auge 30 erzeugte Lichtmuster 40. Als Teile des Auges 30 sind wiederum die Sklera 31, die Iris 32 und die Pupille 33 gezeigt. Die Lichtmuster 40 entstehen durch Lichtreflexe auf der Kornea des Auges 30.

Das in **Figur 2a** gezeigte Lichtmuster 40 besteht aus einer ellipsenähnlichen Anordnung von Lichtpunkten. Die Lichtpunkte oder kornealen Lichtreflexe werden in diesem Beispiel durch eine kreisrunde Anordnung von LEDs mit vorgeschalteten Diffusorelementen als Beleuchtungseinrichtung 12 der Vorrichtung 10 erzeugt (siehe Fig. 1). Die Verformung zu einer ellipsenähnlichen Anordnung ergibt sich aus dem Astigmatismus des Auges 30.

Die Längsachse der Ellipse entspricht etwa der Astigmatismusachse 34 des Auges 30.

In **Figur 2b** wird zur Beleuchtung des Auges 30 eine ringförmige bzw. kreisrunde, diffuse Lichtquelle als Beleuchtungseinrichtung 12 (siehe Fig. 1) verwendet. Als Lichtmuster 40 ergibt sich ein ellipsenähnlicher Lichtreflex auf der Kornea des Auges 30. Auch in diesem Beispiel entsteht die ellipsenähnliche Form des Lichtmusters 40 aus dem Astigmatismus des Auges 30, wobei die große Halbachse der Ellipse etwa der Astigmatismusachse 34 des Auges 30 entspricht und deren Richtung angibt.

In **Figur 2c** wird zur Beleuchtung des Auges 30 eine Anordnung von mehreren ringförmigen, diffusen Lichtquellen, die als konzentrische Kreise angeordnet sind, als Beleuchtungseinrichtung 12 verwendet. Als Lichtmuster 40 ergeben sich ellipsenhafte Lichtreflexe auf der Kornea des Auges 30. Auch in diesem Beispiel entsteht die ellipsenhafte Form des Lichtmusters 40 aus dem Astigmatismus des Auges 30, wobei die großen Halbachsen der Ellipsen etwa der Astigmatismusachse 34 des Auges 30 entsprechen und deren Richtung angeben.

Gemäß der Erfindung ist es nicht notwendig die Torsion, also die relative Verdrehung des Auges zwischen Diagnose und Operation, durch einen bildmerkmalbasierten Vergleich zwischen einem Diagnose- und einem Operationsbild indirekt durch Mittel der Bildverarbeitung zu bestimmen. Stattdessen werden relevante Parameter des Auges, wie zum Beispiel die Astigmatismusachse, direkt während der Operation durch beispielsweise topometrische, topographische, keratometrische, keratographische oder ophthalmometrische Messungen bestimmt. Damit wird man vom Bildmaterial aus der Diagnose unabhängig.

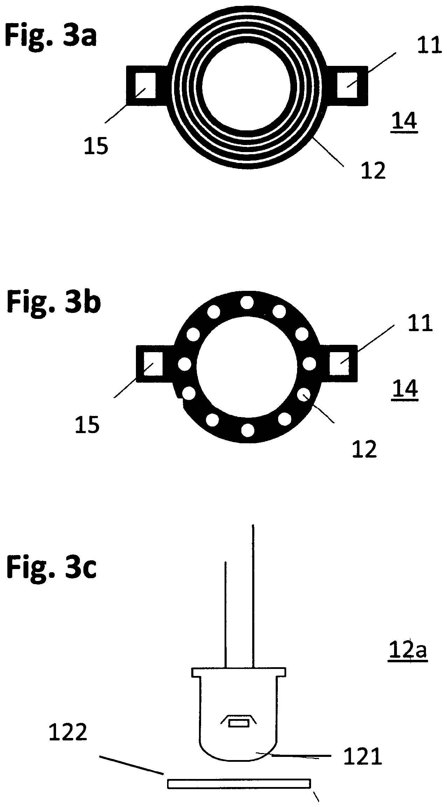

Die **Figuren 3a und 3b** zeigen in einer Draufsicht von unten, das heißt vom Operationsobjekt bzw. Patientenauge aus gesehen, eine Befestigungseinrichtung 14 mit der Kamera 11, der Beleuchtungseinrichtung 12 zur Erzeugung des Lichtmusters 40 und der zweiten Beleuchtungseinrichtung 15. Die Beleuchtungseinrichtung 12 umfasst eine ringförmige Anordnung von diffusen oder afokalen Lichtquellen. Die Kamera 11 ist optional, für den Fall, dass nicht die integrierte Kamera des Operationsmikroskops verwendet wird. Als Beleuchtungseinrichtung 12 sind verschiedenartige Lichtquellenanordnungen zur Erzeugung des Lichtmusters auf dem Auge 30 gezeigt.

Die Lichtquellen der Beleuchtungseinrichtung 12 erzeugen diffuses oder afokales Licht. Die Halterung 14 (siehe Fig. 1) kann zu diesem Zweck mit einer Anzahl von LEDs mit vorgesetzten Diffusorelementen bestückt sein (Fig. 3b) oder z.B. mit diffus leuchtenden Ringen, die als konzentrische Kreise ausgebildet sind (Fig. 3a). Es ist aber auch möglich statt der leuchtenden LEDs oder Ringe helle, nicht leuchtende Punkte oder Kreise zu benutzen, die mit einer sekundären Lichtquelle angeleuchtet werden.

**Figur 3c** zeigt als Beispiel eine diffuse Lichtquelle 12a der Beleuchtungseinrichtung 12 mit einer LED 121 und einem vor der LED 121 als Vorsatz angeordneten Diffusorelement 122, das als lichtstreuende Folie oder Glasscheibe ausgestaltet ist.

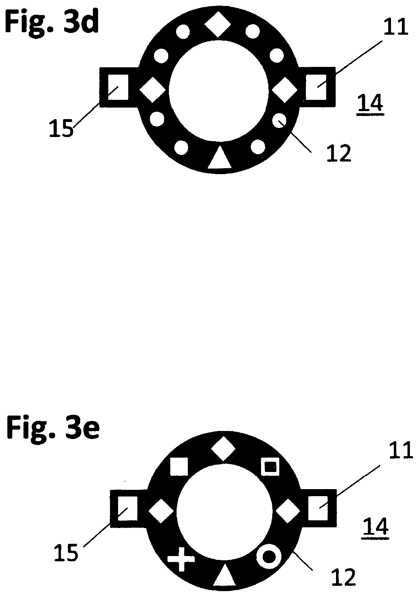

**Figuren 3d und 3e** zeigen als weitere Beispiele die Befestigungseinrichtung 14 wie in den Figuren 3a und 3b bereits beschrieben, wobei die Beleuchtungseinrichtung 12 zur Erzeugung spezifischer Reflexformen auf dem Auge ausgestaltet ist. Dazu haben einzelne Lichtelemente oder Lichtquellen 12a der Beleuchtungseinrichtung 12 eine spezielle geometrische Form, die derart gestaltet ist, dass sich der durch sie auf dem Auge erzeugte Lichtreflex von anderen Lichtreflexen oder Fremdreflexen unterscheidet.

In den gezeigten Beispielen sind die einzelnen Lichtquellen der Beleuchtungseinrichtung 12 rechteckig, quadratisch, rautenförmig oder auch ringförmig gestaltet, wobei die Lichtquellen mit ihren unterschiedlichen geometrischen Formen in definierter Anordnung vorgesehen sind. Die hierdurch auf dem Auge erzeugten Lichtreflexe sind sehr gut zu erkennen und sehr gut von Lichtreflexen auf dem Auge unterscheidbar, die von anderen Lichtquellen stammen.

Optional oder zusätzlich können die einzelnen Lichtquellen der Beleuchtungseinrichtung 12 auch gepulst betrieben werden oder Licht bestimmter oder verschiedener Wellenlängen emittieren, um eine spezifische Reflexform bzw. einen spezifischen Lichtreflex auf dem Auge zu erzeugen, der von anderen Lichtreflexen auf dem Auge unterschieden werden kann.

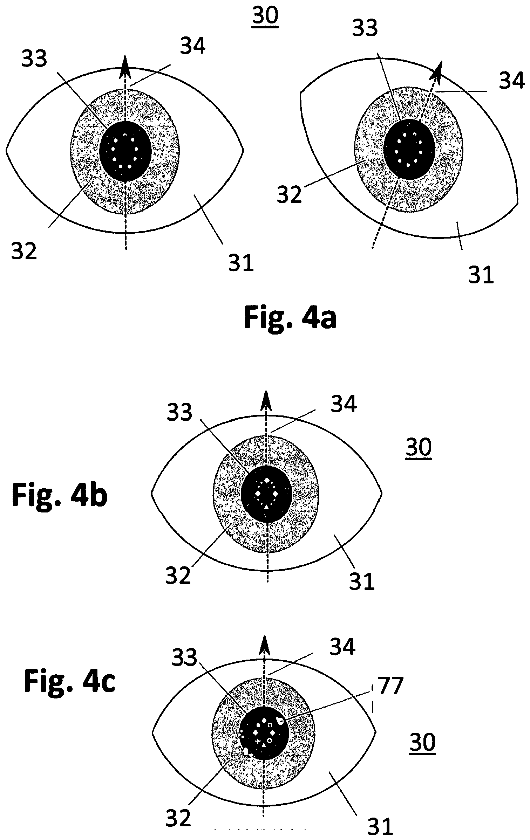

**Figur 4a** zeigt als Beispiel Bilder des Auges 30 bei der Diagnose (linkes Bild) und während der Operation (rechtes Bild), wobei als Beleuchtungseinrichtung 12 (siehe Fig. 1) ein mit LEDs und vorgesetzten Diffusorelementen bestückter Ring verwendet wird, wie er in Figur 3b gezeigt und weiter oben beschrieben wurde. Die Bezugszeichen kennzeichnen jeweils dieselben Elemente des Auges 30 wie in den vorhergehenden Figuren. In diesem Beispiel hat sich die Lage der gemessenen Astigmatismusachse 34 zum Zeitpunkt der Operation gegenüber ihrer Lage zum Zeitpunkt der Diagnose geändert. Diese Änderung wird als statische Torsion bezeichnet. Fehler aufgrund dieser Torsion werden durch die Erfindung vermieden.

Die **Figuren 4b und 4c** zeigen als weitere Beispiele Bilder des Auges 30 mit Lichtreflexen, die spezifische Reflexformen auf dem Auge aufweisen. Gleiche Bezugszeichen wie in den vorherigen Figuren kennzeichnen dieselben Elemente und wurden oben bereits beschrieben. Die in Figur 4b dargestellte Reflexform wird durch die in Figur 3d gezeigte Beleuchtungseinrichtung 12 erzeugt, während die in Figur 4c dargestellte Reflexform durch die in Figur 3e gezeigte Beleuchtungseinrichtung erzeugt wird. Zur Veranschaulichung sind in Figur 4c zusätzlich Fremdreflexe 77 gezeigt, die aufgrund der spezifischen Reflexform der Beleuchtungseinrichtung sehr gut als solche erkennbar sind.

Die ermittelte Lage der Astigmatismusachse 34 während der Operation kann jedoch optional auch mit der Achslage während der Diagnose verglichen werden, die vorzugsweise nach dem gleichen Prinzip oder auch mit einem anderen Verfahren ermittelt wurde.

Das Messprinzip während Diagnose und während der Operation muss nicht notwendigerweise identisch sein. Allerdings müssen jeweils Parameter ableitbar sein, die man auf ihre räumliche Lage hin vergleichen kann. Beispielsweise kann während der Diagnose eine topographische Messung mit sogenannten Placido-Ringen durchgeführt werden, aus der zunächst die ortsaufgelöste Oberflächenkrümmung und anschließend sowohl die Augenoberfläche selbst als auch die Astigmatismusachse berechnet werden kann. Andererseits kann während der Operation die Augenoberfläche z.B. durch Streifenprojektionstechniken als Punktewolke gemessen werden. Auch hieraus lässt sich die Astigmatismusachse 34 ableiten. Man kann nun entweder, wie oben bereits erwähnt, die 3D-Oberflächenpunktewolken mittels Registrierung miteinander vergleichen oder auch nur die Orientierung der daraus jeweils ableitbaren Astigmatismusachse, um die Torsion zwischen Diagnose und Operation zu ermitteln.

Es ist aber auch möglich, die Augenoberfläche während der Diagnose und der Operation direkt zu vermessen. Hier können insbesondere auch Triangulationsverfahren, Streifenprojektionstechniken, Stereovision oder ähnliche Verfahren angewendet werden. Im Ergebnis wird eine 3D-Punktewolke generiert, die die Augenoberfläche repräsentiert. Die bei Diagnose und Operation anfallenden Punktewolken können miteinander verglichen werden. Aus diesem Registrierprozess lässt sich u.a. ebenfalls die Torsion berechnen.

Technisch am einfachsten ist es jedoch, wenn während der Operation das gleiche Messverfahren zur Anwendung kommt wie bei der Diagnose. Wenn z.B. während der Diagnose eine ringartige Anordnung von LEDs mit vorgeschalteten Diffusorelementen für eine Astigmatismusmessung benutzt wird, dann kann während der Operation ebenfalls eine solche Anordnung verwendet werden. Die jeweils ermittelten Astigmatismusachsen können anschließend verglichen werden. Die Torsion ergibt sich aus dem von diesen Achsen eingeschlossenen Winkel.

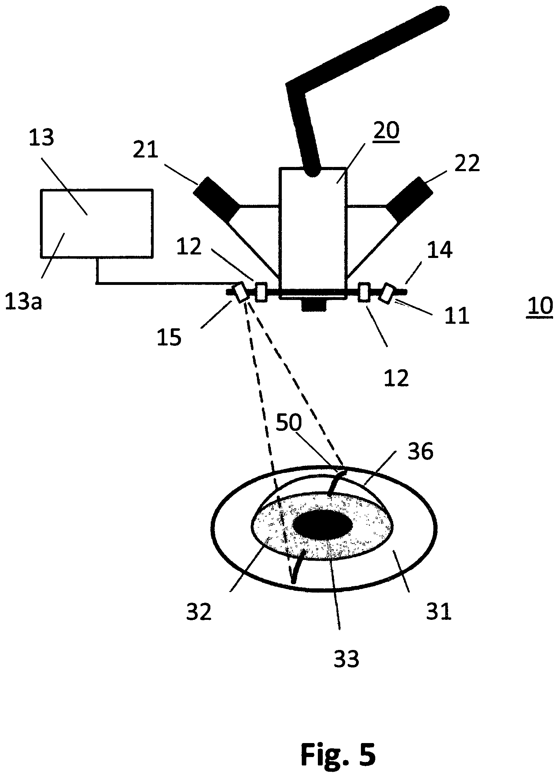

**Figur 5** zeigt ein Operationsmikroskop 20, das die erfindungsgemäße Vorrichtung 10 umfasst. Die Elemente des Operationsmikroskops 20 einschließlich der darin integrierten oder daran abnehmbar befestigten Vorrichtung 10 sind mit denselben Bezugszeichen versehen wie in Figur 1. Auf dem Auge 30 wird durch die zweite Beleuchtungseinrichtung 15 eine Lichtmarkierung 50 erzeugt. Die Lage und Ausrichtung der Lichtmarkierung 50 auf dem Auge 30 wird aufgrund des von der Recheneinheit 13 aus Bilddaten der Kamera 11 oder einer integrierten Mikroskopkamera ermittelten Parameters gesteuert.

Hierfür dient die Recheneinheit als Steuereinheit 13a bzw. hat eine solche integriert. In diesem Beispiel wird die während der Operation aus dem Lichtmuster 40 (siehe Figuren 2a bis 2c) ermittelte Astigmatismusachse 34 des Auges 30 durch die Lichtmarkierung 50 auf dem Auge 30 angezeigt und ist somit für den Arzt auf dem Auge 30 sichtbar.

Die Elemente des Auges 30 sind wie oben beschrieben und mit denselben Bezugszeichen versehen. Die Datenverbindung zwischen der Kamera 11 und der Rechen- bzw. Steuereinheit 13 bzw. 13a ist in Figur 5 der Übersichtlichkeit halber nicht dargestellt.

Durch die zweite Beleuchtungseinrichtung 15 in Form eines Projektors oder eines Lasers, wie zum Beispiel eines Linien- oder Punktlasers, wird die Lage der Astigmatismusachse 34 oder die Sollorientierung einer einzusetzenden Intraokularlinse direkt auf das Auge projiziert und somit dem Arzt direkt auf dem Auge angezeigt.

Die Recheneinheit 13 wertet hierzu die Kamerabilder des Mikroskops aus und weist den Projektor bzw. einen Mustergenerator an, z.B. eine Linie als Lichtmarkierung 50 zu generieren, die z.B. die Astigmatismusachse 34 des Auges 30 oder eine zu dieser in definierter Beziehung stehender Achse anzeigt. Auch andere Informationen sind sinnvoll, wie beispielsweise die Orte, an denen Inzisionen durchgeführt werden sollen. Deren Position wird durch die Auswertung der von den Leuchtquellen bzw. der Beleuchtungseinrichtung 12 auf der Hornhaut erzeugten Reflexe mitbestimmt.

Die Recheneinheit 13 kann zusätzlich das Auge 30 tracken bzw. verfolgen, damit der Projektor oder Laser die Linie immer an der gleichen Stelle generieren kann, z.B. immer limbusmittig. Das heißt, die Recheneinheit 13 kann zeitgleich das Auge, z.B. die Pupille oder den Limbus, verfolgen und den Projektor bzw. die zweite Beleuchtungseinrichtung 15 anweisen, die Sollachse, sichtbar für den Arzt, pupillenmittig oder limbusmittig zu projizieren.

Trackingalgorithmen können die aktuelle Augenposition inklusive der Torsion ermitteln und so die Projektion z.B. einer mitwandemden Sollachse der Intraokularlinse auf das Auge ermöglichen. Auch ist die Anzeige weiterer Informationen möglich, wie z.B. die Orte, an denen die Inzisionen gemacht werden sollen, der Limbusrand, die optische und visuelle Achse des Patienten, die Position der Kapsulorhexis, usw.

Bei Verwendung eines Projektors oder generell eines Mustergenerators als Beleuchtungseinrichtung 15, der für die Visualisierung z.B. der Astigmatismusachse vorgesehen ist, kann man bei Bedarf mit Hilfe der Kamera 11 oder der internen Mikroskopkamera, welche einen Winkel mit der Projektions- bzw. Beleuchtungseinrichtung 15 einschließt, auch 3D-Daten generieren. Mit Hilfe von speziellen statischen oder bewegten Mustern wird in der Recheneinheit 13 eine Punktewolke generiert, die die Augenoberfläche oder Teile davon repräsentiert. Dadurch ist es beispielsweise möglich, die Blickrichtung des Auges 30 zu bestimmen. Falls eine gewisse Asphärizität der Augenoberfläche oder Teilen davon gegeben ist, kann auf diese Weise auch die dynamische oder intraoperative Torsion bestimmt werden. Es ist ebenso möglich, die Ausrichtung von Operationsinstrumenten zu ermitteln, die sich im Sichtfeld des Musters und der Kamera 11 befinden.

Bei der Erhebung der intraoperativen Augendaten beispielsweise mit Hilfe der Reflexe oder mit Hilfe des oben erwähnten Mustergenerators entstehen bei entsprechendem Einfallswinkel auch Reflexe auf der Intraokularlinse, welche für eine Lagebestimmung benutzt werden können. Damit kann man z.B. die Achse einer torischen Linse mit der Astigmatismusachse der Kornea vergleichen und so eine eventuelle Fehlorientierung der Intraokularlinse ermitteln. Ebenso kann eine Schräglage der Linse detektiert werden.

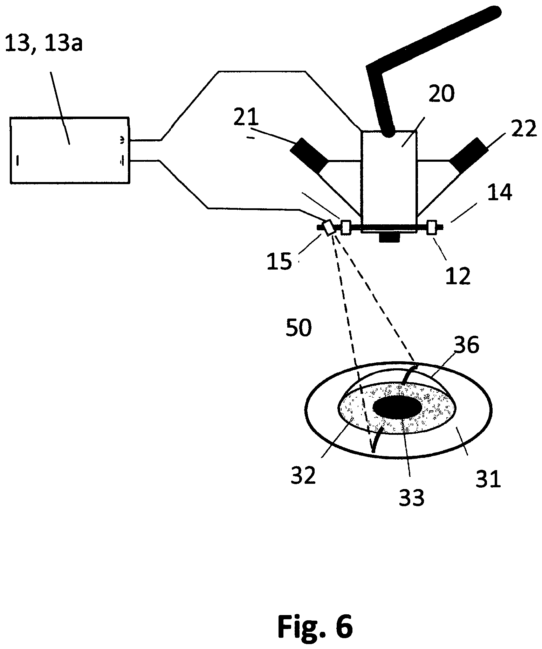

**Figur 6** zeigt ein erfindungsgemäßes Operationsmikroskop 20, bei dem als Kamera 11 zur Erfassung des auf dem Auge erzeugten Lichtmusters die interne Kamera des Operationsmikroskops 20 verwendet wird. Die mit den weiteren Bezugszeichen gekennzeichneten Elemente wurden oben bereits beschrieben.

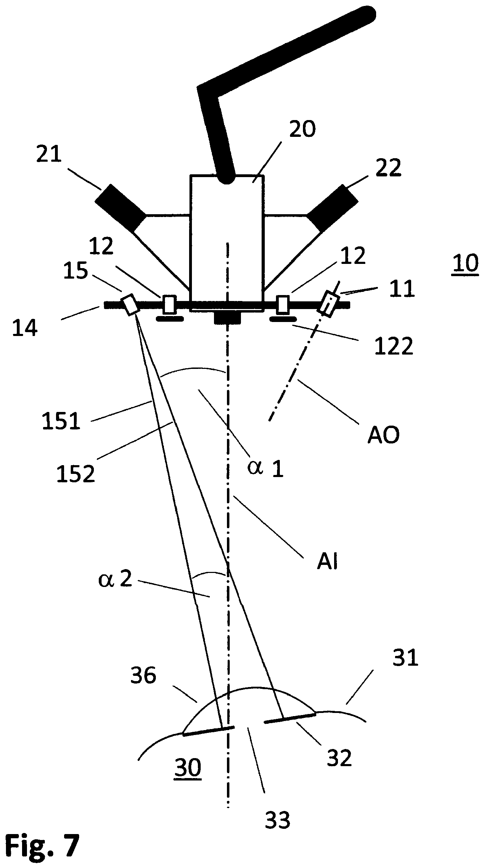

**Figur 7** zeigt ein Operationsmikroskop 20, das die erfindungsgemäße Vorrichtung 10 umfasst, wie mit Bezug auf die Figuren 1 und 5 bereits beschrieben. Die Recheneinheit 13 (s. Figur 5) ist der Übersichtlichkeit halber in dieser Figur nicht dargestellt. Die dargestellten Elemente sind mit denselben Bezugszeichen versehen wie in Figuren 1 und 5. Zusätzlich sind Diffusorelemente 122 explizit dargestellt, die den LEDs der ersten Beleuchtungseinrichtung 12 vorgeschaltet sind. Die Kamera 11 ist optional, für den Fall dass nicht die integrierte Kamera des Operationsmikroskops zur Erfassung der Lichtmuster auf dem Auge 30 verwendet wird. Sie kann aber auch zusätzlich zu einer integrierten Kamera des Operationsmikroskops verwendet werden.

Die zweite Beleuchtungseinrichtung 15 in Form eines Mustergenerators erzeugt in dem hier gezeigten Beispiel ein Linienpaar 151, 152, das auf das Auge 30 projiziert wird. Die einzelnen Linien 151, 152 schließen die Winkel α1 bzw. α2 mit der optischen Achse AI der internen Kamera des Operationsmikroskops 20 ein. Die Größenordnung dieser Winkel beträgt vorteilhafterweise etwa 5 bis 45 Grad. Die Winkel, die die Linien 151, 152 mit der optischen Achse der optionalen Kamera 11 einschließen, sind der Übersicht halber nicht dargestellt.

Die Tatsache, dass die Winkel α1 bzw. α2 größer als Null sind, ermöglicht es die Ausrichtung bzw. Verkippung des Auges 30 zu bestimmen. Je größer die Verkippung oder Rotation des Auges um seine horizontale und vertikale Achse ist, desto weniger koinzidiert das Zentrum der Reflexe mit dem Apex der Kornea 36. Dadurch beziehen sich die Aussagen aus den auf den Reflexmustern basierenden Rechnungen nicht mehr auf den kornealen Apex. Die berechnete Astigmatismusachse kann je nach Verkippung von der zentralen Astigmatismusachse abweichen und ist deshalb nur noch bedingt mit der Astigmatismusachse während der Diagnose vergleichbar.

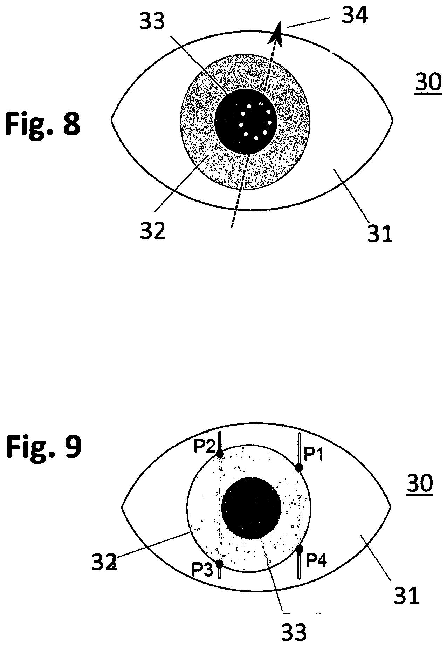

Eine Augenverkippung von beispielsweise 5° ergibt bereits eine Dezentrierung des Lichtreflexzentrums vom Apex von 1mm. Bei 10° steigt dieser Wert auf ca. 2mm. Die Dezentrierung ist zur Veranschaulichung in **Figur 8** dargestellt, wobei in dem gezeigten Beispiel das Auge 30 nach rechts rotiert ist. Die verwendeten Bezugszeichnen kennzeichnen die Elemente des Auges 30 wie sie oben bereits beschrieben wurden.

Die Tatsache, dass im Kamerabild das Zentrum der Reflexe und das Pupillen- oder Limbuszentrum nicht übereinstimmen, kann nur bedingt zur Feststellung einer Verkippung verwendet werden, da bei asymmetrisch geformtem Limbus oder Pupille auch bei verschwindender Verkippung nicht notwendigerweise eine Koinzidenz von Reflexzentrum und Pupillen- oder Limbuszentrum bestehen muss. Es ist auch möglich, dass der Limbus partiell derart unscharf ist, dass das Limbuszentrum nicht mehr zuverlässig erkannt bzw. bestimmt werden kann.

Man kann die Verkippung detektieren, indem man beispielsweise mit Hilfe eines Mustergenerators zu beliebigen Zeitpunkten ein Lichtmuster auf dem Auge erzeugt. Dies ist als Beispiel in **Figur 9** gezeigt. Die verwendeten Bezugszeichnen kennzeichnen die Elemente des Auges 30 wie sie oben bereits beschrieben wurden.

Zur Bestimmung der Verkippung des Auges 30 werden die Schnittpunkte P1, P2, P3, P4 des Lichtmusters, das in dem dargestellten Beispiel ein Linienpaar ist und wie in Figur 7 gezeigt auf das Auge 30 projiziert wird, mit dem Limbus ermittelt. Anschließend werden nach einem vorangegangenen, patientenunabhängigen, Kalibrierprozess die 3d-Koordinaten dieser Schnittpunkte P1, P2, P3, P4 berechnet, mit deren Hilfe eine Ausgleichsebene approximiert wird. Die Ebenennormale gibt schließlich die Augenausrichtung an. Das gezeigt Linienpaar ist nur ein Beispiel von vielen möglichen Mustern. Es muss jedoch mindestens drei nicht auf einer Linie liegenden Punkte geben.

Es ist auch möglich, die Anzeige der Astigmatismusachse 34 und die Berechnung der Verkippung zu kombinieren, indem beispielsweise ein relativ enges Linienpaar auf dem Auge 30 generiert wird, das einerseits eine Verkippung ermittelt und andererseits die ermittelte Astigmatismusachse anzeigt. Insbesondere eine spezifische Farbe oder ein Blinken der auf dem Auge generierten Linien liefert dem Arzt Hinweise auf die momentane Verkippung.

Alternativ kann die Augenausrichtung auch ohne einen Mustergenerator mit Hilfe einer zusätzlichen Kamera mittels Stereovision ermittelt werden. Dazu wird beispielsweise der Limbusrand in beiden Kamerabildern detektiert, um anschließend die jeweiligen Bildkoordinaten zur Berechnung seiner räumlichen Lage zu benutzen.

Ein wesentlicher Vorteil des erfindungsgemäßen System und Verfahrens ist die Tatsache, dass man durch die Neumessung von Augenparametern, wie z.B. der Astigmatismusachse, nicht auf Bildmaterial aus der Diagnose angewiesen ist.

Insbesondere können Intraokularlinsen mit hoher Genauigkeit in der vorgesehenen Position, zum Beispiel in Bezug auf die Achslage des Auges, in das Auge eingebracht werden, ohne dass eine Torsion des Auges während der Operation oder zwischen Diagnose- und Operationszeitpunkt die Genauigkeit beeinträchtigt. Dabei kann die Linsenachse an der für den Arzt während der Operation sichtbaren Achslage des Auges orientiert werden.

Bei Laserbehandlungen kann insbesondere die die statische Torsion sehr gut gemessen werden. Auch können zum Beispiel Schussmuster bei Laserbehandlungen entsprechend der gemessenen intraoperativen Achslage vor Beginn der Behandlung so gedreht werden, dass die Behandlungsfehler durch die statische Torsion vermieden werden.

## Patentansprüche

1. Vorrichtung zur Bestimmung der Ausrichtung des Auges bei Augenoperationen, umfassend:
eine Beleuchtungseinrichtung (12) zur Beleuchtung des Auges eines sich in der Operationslage befindlichen Patienten;
eine Kameraeinrichtung (11), um ein Bild des Auges während einer Augenoperation zu erfassen; und
eine Recheneinheit (13);
**dadurch gekennzeichnet, dass**
die Beleuchtungseinrichtung (12) eine diffuse Lichtquelle oder eine Anordnung von mehreren diffusen Lichtquellen umfasst, deren einzelne Lichtelemente oder Lichtquellen zur Erzeugung spezifischer Reflexformen auf dem Auge und mit geometrischen Formen in definierter Anordnung vorgesehen sind, und die Beleuchtungseinrichtung derart ausgebildet ist, dass sie auf der Kornea des Auges (30) des sich in Operationslage befindlichen Patienten Lichtreflexe erzeugt, die ein Lichtmuster (40) bilden, welches die spezifischen Reflexformen umfasst,
wobei das Lichtmuster (40) von der Kameraeinrichtung (11) während der Augenoperation erfasst wird:
und
die Recheneinheit (13) derart ausgestaltet ist, dass sie während der Augenoperation aus dem erfassten Lichtmuster (40) aufgrund der spezifischen Reflexformen mindestens eine Eigenschaft des Auges (30) ermittelt, die von der momentanen Ausrichtung des Auges (30) abhängig ist.

2. Vorrichtung nach Anspruch 1, **gekennzeichnet durch** eine Befestigungseinrichtung (14) zur Befestigung der Beleuchtungseinrichtung (12) und/oder der Kameraeinrichtung (11) an einem Operationsmikroskop (20).

3. Vorrichtung nach Anspruch 1 oder 2, **gekennzeichnet durch** eine Steuereinheit (13a), die zur Steuerung einer zweiten Beleuchtungseinrichtung (15) zur Erzeugung einer Lichtmarkierung (50) auf dem Auge (30) aufgrund der aus dem Lichtmuster (40) momentan ermittelten Eigenschaft des Auges (30) während der Augenoperation und/oder zur Steuerung eines Ablationslasers aufgrund der momentan ermittelten Eigenschaft des Auges (30) während der Augenoperation ausgestaltet ist.

4. Vorrichtung nach einem der vorhergehenden Ansprüche, **dadurch gekennzeichnet, dass** die aus dem Lichtmuster (40) ermittelte Eigenschaft eine spezifische lageabhängige Charakteristik des Auges (30) und/oder eine spezifische topografische Eigenschaft des Auges (30) und/oder die Lage der Astigmatismusachse (34) oder eine hierzu in definierter Beziehung stehende Richtung kennzeichnet.

5. Vorrichtung nach einem der vorhergehenden Ansprüche, **gekennzeichnet durch** eine zweite Beleuchtungseinrichtung (15) zur Erzeugung einer Lichtmarkierung (50) auf dem Auge (30), die während der Augenoperation auf dem Auge (30) ein oder mehrere der folgenden Informationen darstellt:
a) eine für die Operation benötigte oder hilfreiche Ortsmarkierung;
b) die Soll-Ausrichtung einer in das Auge (30) einzusetzenden Intraokularlinse;
c) die Soll-Position eines durchzuführenden Schnittes;
d) die Lage von Limbus und/oder Pupille;
e) die Richtung der Astigmatismusachse oder eine hierzu in definierter Beziehung stehende Richtung.

6. Vorrichtung nach einem der vorhergehenden Ansprüche, **dadurch gekennzeichnet, dass** die Beleuchtungseinrichtung (12) zur Erzeugung des Lichtmusters (40) diffuses oder afokales Licht erzeugt.

7. Vorrichtung nach einem der vorhergehenden Ansprüche 5 oder 6, **dadurch gekennzeichnet, dass** die zweite Beleuchtungseinrichtung (15) derart angeordnet ist, dass das Licht zur Erzeugung der Lichtmarkierung (50) unter einem Winkel α zur Achse der Erfassung des Lichtmusters (40) auf dem Auge (30) einfällt.

8. Vorrichtung nach einem der vorhergehenden Ansprüche, **dadurch gekennzeichnet, dass** die Beleuchtungseinrichtung (12) derart ausgestaltet ist, dass auf dem Auge spezifische Reflexformen erzeugt werden.

9. Operationsmikroskop für Augenoperationen, **gekennzeichnet durch** eine Vorrichtung nach einem der Ansprüche 1 bis 8.

10. Computerprogramm zur Steuerung eines Augenchirurgiesystems, das zur Durchführung der folgenden Schritte mittels einer Vorrichtung nach einem der Ansprüche 1 bis 8 ausgestaltet ist:
Beleuchten eines Auges (30) und Erfassen eines Bildes des Auges (30);
**dadurch gekennzeichnet, dass**
die Gestalt einer speziell gestalteten diffusen Lichtquelle oder Anordnung von mehreren diffusen Lichtquellen, deren einzelne Lichtelemente oder Lichtquellen zur Erzeugung spezifischer Reflexformen auf dem Auge und mit geometrischen Formen in definierter Anordnung vorgesehen sind, nach Reflexion an der Kornea des Auges (30) des sich in Operationslage befindlichen Patienten als
ein Lichtmuster (40), welches die spezifischen Reflexformen umfasst, erfasst wird,
und während der Augenoperation aus dem erfassten Lichtmuster (40) aufgrund der spezifischen Reflexformen mindestens eine Eigenschaft des Auges (30) bestimmt wird, die die momentane Ausrichtung des Auges (30) kennzeichnet.

11. Computerpogramm nach Anspruch 10, **dadurch gekennzeichnet, dass** die aus dem Lichtmuster (40) bestimmte Eigenschaft zur Steuerung einer auf dem Auge (30) erzeugten Lichtmarkierung (50) und/oder zur Korrektur der Position der Ablationsorte bei einer Laserbehandlung des Auges (30) in Abhängigkeit von der momentanen Ausrichtung des Auges (30) und/oder zur Bestimmung der Blickrichtung verwendet wird.

12. Computerpogramm nach Anspruch 11, **dadurch gekennzeichnet, dass** die aus dem Lichtmuster (40) ermittelte Eigenschaft eine spezifische topografische Eigenschaft des Auges (30) ist.

13. Computerpogramm nach Anspruch 11 oder 12, **dadurch gekennzeichnet, dass** die aus dem Lichtmuster (40) ermittelte Eigenschaft die Lage der Astigmatismusachse des Auges (30) und/oder eine hierzu in definierter Beziehung stehende Richtung kennzeichnet.

14. Computerpogramm nach einem der Ansprüche 11 bis 13, **dadurch gekennzeichnet, dass** die aus dem Lichtmuster (40) ermittelte Eigenschaft durch Erhebung von Daten des Auges (40) während der Operation bestimmt wird, wobei die Daten insbesondere aus topometrischen, topografischen, keratometrischen, keratografischen oder ophthalmometrischen Messungen gewonnen werden.

15. Computerpogramm nach Anspruch 14, **dadurch gekennzeichnet, dass** die momentanen Daten oder daraus ableitbare Größen mit entsprechenden während der Diagnose ermittelten Daten oder daraus ableitbaren Größen verglichen werden, um daraus eine Lageveränderung während der Operation gegenüber dem Diagnosezeitpunkt zu bestimmen.

16. Computerpogramm nach Anspruch 14 oder 15, **dadurch gekennzeichnet, dass** die während der Operation erhobenen Daten und die zugehörigen Bilder gespeichert werden und als Referenzdaten oder -bilder für nachfolgende bildverarbeitungsbasierte Schritte verwendet werden.

17. Computerpogramm nach einem der Ansprüche 10 bis 16, **dadurch gekennzeichnet, dass** das Lichtmuster (40) auf dem Auge (30) mit einer diffusen oder afokalen Beleuchtungsquelle erzeugt wird.

18. Computerpogramm nach einem der Ansprüche 11 bis 17, **dadurch gekennzeichnet, dass** die Lichtmarkierung (50) während der Augenoperation auf dem Auge (30) ein oder mehrere der folgenden Informationen anzeigt:
a) eine für die Operation benötigte oder hilfreiche Ortsmarkierung;
b) die Soll-Ausrichtung einer in das Auge (30) einzusetzenden Intraokularlinse;
c) die Soll-Position eines durchzuführenden Schnittes;
d) die Lage von Limbus und/oder Pupille;
e) die Richtung der Astigmatismusachse oder eine hierzu in definierter Beziehung stehende Richtung.

19. Computerpogramm nach einem der Ansprüche 11 bis 18, **dadurch gekennzeichnet, dass** Licht zur Erzeugung der Lichtmarkierung (50) auf dem Auge (30) unter einem Winkel α zur Achse (AI, AO) der Erfassung des Lichtmusters (40) auf dem Auge (30) einfällt.

20. Steuereinheit für Augenchirurgiesysteme, die ein Computerpogramm nach einem der Ansprüche 10 bis 19 umfasst.

## Claims

1. Apparatus for determining the orientation of the eye during eye surgeries, comprising:
an illumination device (12) for illuminating the eye of a patient in the operation position;
a camera device (11) for capturing an image of the eye during an eye surgery; and
a computing unit (13);
**characterized in that**
the illumination device (12) comprises a diffuse light source or an arrangement of several diffuse light sources, the individual light elements or light sources of which are provided for producing specific reflection shapes on the eye and with geometric shapes in a defined arrangement,
and the illumination device is designed in such a way that it generates light reflections on the cornea of the eye (30) of the patient in the operating position, which form a light pattern (40) comprising the specific reflection shapes,
wherein the light pattern (40) is detected by the camera device (11) during the eye surgery,
and the computing unit (13) is configured in such a way that during the eye surgery it determines from the detected light pattern (40), on the basis of the specific reflection shapes, at least one property of the eye (30) which is dependent on the instantaneous orientation of the eye (30).

2. Apparatus according to claim 1, **characterised by** a fastening device (14) for fastening the illumination device (12) and/or the camera device (11) to a surgical microscope (20).

3. Apparatus according to claim 1 or 2, **characterised by** a control unit (13a) which is designed for controlling a second illumination device (15) for generating a light mark (50) on the eye (30) on the basis of the property of the eye (30) currently determined from the light pattern (40) during the eye surgery and/or for controlling an ablation laser on the basis of the property of the eye (30) currently determined during the eye surgery.

4. Apparatus according to one of the preceding claims, **characterised in that** the property determined from the light pattern (40) characterizes a specific position-dependent characteristic of the eye (30) and/or a specific topographical property of the eye (30) and/or the position of the astigmatism axis (34) or a direction having a defined relationship thereto.

5. Apparatus according to any one of the preceding claims, **characterised by** a second illumination device (15) for generating a light mark (50) on the eye (30) representing one or more of the following information on the eye (30) during the eye surgery:
a) a location marker required or helpful for the surgery;
b) the desired orientation of an intraocular lens to be inserted in the eye (30);
c) the desired position of an incision to be made;
d) the position of the limbus and/or pupil;
e) the direction of the axis of astigmatism or a direction in a defined relationship thereto.

6. Apparatus according to any one of the preceding claims, **characterised in that** the illumination device (12) for generating the light pattern (40) generates diffuse or afocal light.

7. Apparatus according to any one of the preceding claims 5 or 6, **characterized in that** the second illumination device (15) is arranged in such a way that the light for producing the light mark (50) is incident on the eye (30) at an angle α with respect to the axis of detection of the light pattern (40).

8. Apparatus according to one of the preceding claims, **characterised in that** the illumination device (12) is designed in such a way that specific reflection shapes are produced on the eye.

9. Operation microscope for eye surgeries, **characterised by** a device according to any one of claims 1 to 8.

10. Computer program for controlling an ophthalmic surgical system adapted to perform the following steps by means of an apparatus according to any one of claims 1 to 8:
illuminating an eye (30) and capturing an image of the eye (30);
**characterized in that**
the configuration of a particularly designed diffuse light source or arrangement of several diffuse light sources, the individual light elements or light sources of which are provided for producing specific reflex shapes on the eye and with geometric shapes in a defined arrangement, is detected after reflection on the cornea of the eye (30) of the patient in the operating position as a light pattern (40) which comprises the specific reflex shapes, and during the eye surgery at least one property of the eye (30) is determined from the detected light pattern (40) on the basis of the specific reflex shapes, which property characterizes the instantaneous alignment of the eye (30).

11. Computer program according to claim 10, **characterized in that** the property determined from the light pattern (40) is used to control a light mark (50) produced on the eye (30) and/or to correct the position of the ablation sites during a laser treatment of the eye (30) as a function of the instantaneous orientation of the eye (30) and/or to determine the direction of gaze.

12. Computer program according to claim 11, **characterized in that** the property determined from the light pattern (40) is a specific topographical property of the eye (30).

13. Computer program according to claim 11 or 12, **characterized in that** the property determined from the light pattern (40) indicates the position of the astigmatism axis of the eye (30) and/or a direction having a defined relationship thereto.

14. Computer program according to any one of claims 11 to 13, **characterized in that** the property determined from the light pattern (40) is determined by collecting data from the eye (40) during the surgery, the data being obtained in particular from topometric, topographic, keratometric, keratographic or ophthalmometric measurements.

15. Computer program according to claim 14, **characterised in that** the instantaneous data or variables derivable therefrom are compared with corresponding data determined during the diagnosis or variables derivable therefrom in order to determine therefrom a change in position during the surgery with respect to the time of diagnosis.

16. Computer program according to claim 14 or 15, **characterized in that** the data collected during the surgery and the associated images are stored and used as reference data or images for subsequent image processing-based steps.

17. Computer program according to any one of claims 10 to 16, **characterized in that** the light pattern (40) on the eye (30) is generated with a diffuse or afocal illumination source.

18. Computer program according to any one of claims 11 to 17, **characterized in that** the light mark (50) displays during the eye surgery on the eye (30) one or more of the following information:
a) a location marker required or helpful for the surgery;
b) the desired alignment of an intraocular lens to be inserted in the eye (30);
c) the desired position of an incision to be made;
d) the position of the limbus and/or pupil;
e) the direction of the axis of astigmatism or a direction which is in a defined relationship thereto.

19. Computer program according to any one of claims 11 to 18, **characterized in that** light for generating the light mark (50) on the eye (30) is incident on the eye (30) at an angle α to the axis (Al, AO) of detection of the light pattern (40).

20. Control unit for eye surgery systems comprising a computer program according to any one of claims 10 to 19.

## Revendications

1. Dispositif pour déterminer l'orientation de l'oeil lors d'une opération de l'oeil, comprenant :
un dispositif d'éclairage (12) pour éclairer l'oeil d'un patient en position d'opération,
un dispositif de caméra (11), pour acquérir une image de l'oeil pendant l'opération de l'oeil, et
une unité de calcul (13);
**caractérisé en ce que**
le dispositif d'éclairage (12) comprend une source de lumière diffuse ou un agencement d'une pluralité de sources de lumière diffuses, les éléments de lumière individuels ou les sources de lumière étant prévus pour produire des formes spécifiques de réflexion sur l'oeil et avec des formes géométriques selon un agencement prédéterminé,
et le dispositif d'éclairage est configuré pour produire, sur la cornée de l'oeil (30) du patient en position d'opération, des reflets de lumière qui forment un motif lumineux (40) comprenant les formes spécifiques de réflexion,
le motif lumineux (40) étant acquis par le dispositif de caméra (11) pendant l'opération de l'oeil,
et l'unité de calcul (13) est adaptée pour, pendant l'opération de l'oeil, déterminer au moins une propriété de l'oeil (30) à partir du motif lumineux (40) acquis, sur la base des formes spécifiques de réflexion, laquelle propriété dépendant de l'orientation instantanée de l'oeil (30).

2. Dispositif selon la revendication 1, **caractérisé par** un dispositif de fixation (14) pour fixer le dispositif d'éclairage (12) et/ou le dispositif de caméra (11) à un microscope d'opération (20).

3. Dispositif selon la revendication 1 ou 2, **caractérisé par** une unité de contrôle (13a) adaptée pour contrôler un deuxième dispositif d'éclairage (15) pour produire, sur la base de la propriété de l'oeil (30) déterminée instantanément du motif lumineux (40), un repère lumineux (50) sur l'oeil (30) pendant l'opération de l'oeil
et/ou pour le contrôle d'un laser d'ablation sur la base de la propriété de l'oeil (30) déterminée instantanément pendant l'opération de l'oeil.

4. Dispositif selon l'une des revendications précédentes, **caractérisé en ce que** la propriété déterminée à partir du motif lumineux (40) caractérise une caractéristique dépendant d'une position spécifique de l'oeil (30) et/ou une propriété topographique spécifique de l'oeil (30) et/ou la position de l'axe d'astigmatisme (34) ou une direction ayant un rapport déterminé avec celle-ci.

5. Dispositif selon l'une des revendications précédentes, **caractérisé par** un deuxième dispositif d'éclairage (15) pour produire, pendant l'opération de l'œil, un repère lumineux (50) sur l'œil (30) qui représente une ou plusieurs des informations suivantes :
a) un repère de localisation important ou utile pour l'opération,
b) l'orientation désirée d'une lentille intraoculaire à implanter dans l'oeil,
c) la position désirée d'une incision à effectuer,
d) la position de la limbe et/ou de la pupille,
e) la direction de l'axe d'astigmatisme ou une direction ayant un rapport déterminé avec celle-ci.

6. Dispositif selon l'une des revendications précédentes, **caractérisé en ce que** le dispositif d'éclairage (12) produit une lumière diffuse ou afocale pour produire le motif lumineux (40).

7. Dispositif selon l'une des revendications précédentes 5 ou 6, **caractérisé en ce que** le deuxième dispositif d'éclairage (15) est arrangé pour que la lumière pour produire le repère lumineux (50) est incident sur l'oeil (30) avec un angle α par rapport à l'axe d'acquisition du motif lumineux (40).

8. Dispositif selon l'une des revendications précédentes, **caractérisé en ce que** le dispositif d'éclairage (12) est adapté pour que des formes spécifiques de réflexion soient produites sur l'œil.

9. Microscope d'opération pour des opérations de l'oeil, **caractérisé par** un dispositif selon l'une des revendications 1 à 8.

10. Programme d'ordinateur pour le contrôle d'un système d'opération de l'oeil, adapté pour mener les étapes suivantes au moyen d'un dispositif selon l'une des revendications 1 à 8 :
éclairer un oeil (30) et acquérir une image de l'oeil (30);
**caractérisé en ce que**
la forme d'une source de lumière diffuse spécialement conçue ou l'arrangement d'une pluralité de sources de lumière diffuses, les éléments de lumière individuels ou les sources de lumière étant prévus pour produire des formes spécifiques de réflexion sur l'oeil et avec des formes géométriques selon un agencement prédéterminé, est acquise après réflexion sur la cornée de l'oeil (30) du patient en position d'opération comme un motif lumineux (40) comprenant les formes spécifiques de réflexion,
et, pendant l'opération de l'oeil, au moins une propriété de l'oeil (30) est déterminée à partir du motif lumineux (40) acquis, sur la base des formes spécifiques de réflexion, laquelle propriété caractérise l'orientation instantanée de l'oeil (30).

11. Programme d'ordinateur selon la revendication 10, **caractérisé en ce que** la propriété déterminée à partir du motif lumineux (40) est utilisée pour le contrôle d'un repère lumineux (50) sur l'oeil (30) et/ou pour la correction de la position des zones d'ablation pour un traitement laser de l'oeil (30) en fonction de l'orientation instantanée de l'oeil (30) et/ou pour la détermination de la direction du regard.

12. Programme d'ordinateur selon la revendication 11, **caractérisé en ce que** la propriété déterminée à partir du motif lumineux (40) est une propriété topographique spécifique de l'oeil (30).

13. Programme d'ordinateur selon la revendication 11 ou 12, **caractérisé en ce que** la propriété déterminée à partir du motif lumineux (40) caractérise la position de l'axe d'astigmatisme de l'oeil (30) et/ou une direction ayant un rapport déterminé avec celle-ci.

14. Programme d'ordinateur selon l'une des revendications 11 à 13, **caractérisé en ce que** la propriété déterminée à partir du motif lumineux (40) est déterminée par collecte de données de l'oeil (40) pendant l'opération, les données étant en particulier obtenues par mesures topométriques, topographiques, kératométriques, kératographiques ou ophtalmométriques.

15. Programme d'ordinateur selon la revendication 14, **caractérisé en ce que** les données instantanées ou des valeurs dérivables de celles-ci sont comparées avec des données déterminées par diagnostique correspondantes ou des valeurs dérivables de celles-ci, pour en déterminer, pendant l'opération, un changement de position depuis le moment du diagnostique.

16. Programme d'ordinateur selon la revendication 14 ou 15, **caractérisé en ce que** les données collectées pendant l'opération et les images correspondantes sont enregistrées et sont utilisées comme données ou images de référence pour des étapes subséquentes basées sur un traitement d'images.

17. Programme d'ordinateur selon l'une des revendications 10 à 16, **caractérisé en ce que** le motif lumineux (40) sur l'oeil (30) est produit avec une source de lumière diffuse ou afocale.

18. Programme d'ordinateur selon l'une des revendications 11 à 17, **caractérisé en ce que** le repère lumineux (50) représente sur l'oeil (30), pendant l'opération de l'oeil, une ou plusieurs des informations suivantes :
a) un repère de localisation important ou utile pour l'opération,
b) l'orientation désirée d'une lentille intraoculaire à implanter dans l'oeil,
c) la position désirée d'une incision à effectuer,
d) la position de la limbe et/ou de la pupille,
e) la direction de l'axe d'astigmatisme ou une direction ayant un rapport déterminé avec celle-ci.

19. Programme d'ordinateur selon l'une des revendications 11 à 18, **caractérisé en ce que** de la lumière pour produire le repère lumineux (50) sur l'oeil (30) est incident sur l'oeil (30) avec un angle α par rapport à l'axe (AI, AO) d'acquisition du motif lumineux (40).

20. Unité de contrôle pour un système de chirurgie de l'oeil, comprenant un programme d'ordinateur selon l'une des revendications 10 à 19.
